(19) Europäisches Patentamt
European Patent Office
Office européen des brevets

(11) Veröffentlichungsnummer: **0 198 244**
**A2**

# (12) EUROPÄISCHE PATENTANMELDUNG

(21) Anmeldenummer: **86103540.0**

(22) Anmeldetag: **15.03.86**

(51) Int. Cl.[4]: **C07D 277/82** , C07D 263/58 , A01N 47/34

(30) Priorität: **30.03.85 JP 65019/85**

(43) Veröffentlichungstag der Anmeldung:
**22.10.86 Patentblatt 86/43**

(84) Benannte Vertragsstaaten:
**AT BE CH DE FR GB IT LI NL SE**

(71) Anmelder: **NIHON TOKUSHU NOYAKU SEIZO K.K.**
**No.4, 2-chome, Nihonbashi Honcho Chuo-ku**
**Tokyo 103(JP)**

(72) Erfinder: **Kume, Toyohiko**
**6-7-8, Asahigaoka**
**Hino-shi Tokyo(JP)**
Erfinder: **Tsuboi, Shinichi**
**3-26-1, Hirayama**
**Hino-shi Tokyo(JP)**
Erfinder: **Isono, Kunihiro**
**2-32-4, Asahigaoka**
**Hino-shi Tokyo(JP)**
Erfinder: **Sasaki, Shoko**
**1-7-3, Higashi-Hirayama**
**Hino-shi Tokyo(JP)**
Erfinder: **Hattori, Yumi**
**598, Kobiki-cho**
**Hachioji-shi Tokyo(JP)**

(74) Vertreter: **Schumacher, Günter, Dr. et al**
**c/o Bayer AG Konzernverwaltung RP**
**Patentabteilung**
**D-5090 Leverkusen 1 Bayerwerk(DE)**

(54) **Neue Benzoylharnstoffe.**

(57) Offenbart werden neue Benzoylharnstoffe der Formel (I)

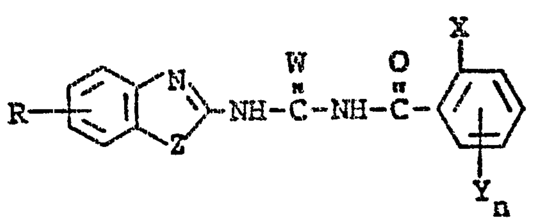

(I)

und die Verwendung der neuen Verbindungen als Insektizide.

EP 0 198 244 A2

Neue Benzoylharnstoffe

Die vorliegende Erfindung betrifft neue Benzoylharnstoffe, ein Verfahren zu ihrer Herstellung sowie die Verwendung dieser Verbindungen als Pestizid, insbesondere als Insektizid.

Es wurde bereits offenbart, daß bestimmte Benzoylharnstoffe insektizide Aktivitäten besitzen - (siehe die JP-OS 124 265/1978).

Nunmehr wurden neue Benzoylharnstoffe der Formel (I)

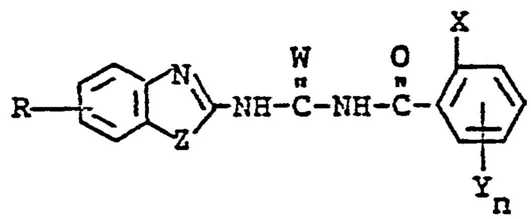

(I)

gefunden, in der

X und Y unabhängig voneindander ein Halogen-Atom oder eine Niederalkyl-Gruppe bezeichnen,

n 0, 1 oder 2 bezeichnet,

Z und W unabhängig voneinander ein Sauerstoff- oder Schwefel-Atom bezeichnen und

R eine Halogen-substituierte Niederalkyl-Gruppe,

eine Halogen-substituierte Niederalkoxy-Gruppe,

eine Halogen-substituierte Niederalkylthio-Gruppe,

eine Halogen-substituierte Niederalkylsulfinyl-Gruppe oder eine Halogen-substituierte Niederalkylsulfonyl-Gruppe bezeichnet.

Die Verbindungen der Formel (I) werden erhalten, wenn die Verbindungen der Formel (II)

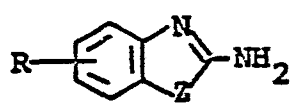

(II)

in der Z und R die angegebenen Bedeutungen haben, mit Verbindungen der Formel (III)

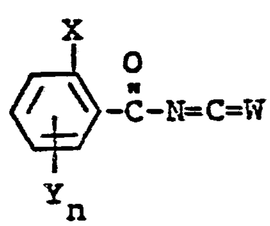

(III)

in der X, Y, n und W die angegebenen Bedeutungen haben, umgesetzt werden, gegebenfalls in Gegenwart inerter Lösungsmittel.

Die neuen Benzoylharnstoffe zeigen hervorragende pestizide Wirkung, insbesondere arthropodizide und besonders bevorzugt insektizide Wirkung.

Überraschenderweise zeigen die Benzoylharnstoffe gemäß der vorliegenden Erfindung eine im wesentlichen stark überlegene insektizide Wirksamkeit, speziell gegen Larven von Insekten der Ordnung Lepidoptera, im Vergleich zu analogen Verbindungen, die aus dem oben zitierten Stand der Technik bekannt sind.

Die Niederalkyl-Gruppen X und Y sind geradkettig oder verzweigt und enthalten 1 bis 8, vorzugsweise 1 bis 6 und besonders bevorzugt 1 bis 4 Kohlenstoff-Atome. Beispiele, die erwähnt werden können, sind Methyl, Ethyl, n-und i-Propyl sowie n-, i-, sec-und tert-Butyl. Bevorzugt werden Methyl und Ethyl, und besonders bevorzugt wird Methyl.

Die Niederalkyl-Gruppen in den Halogen-substituierten Niederalkyl-, Niederalkoxy-, Niederalkylthio-, Niederalkylsulfinyl-oder Niederalkylsulfonyl-Gruppen R sind geradkettig oder verzweigt und enthalten 1 bis 8, vorzugsweise 1 bis 4 und besonders bevorzugt 1 oder 2 Kohlenstoff-Atome. Beispiele, die erwähnt werden können, sind Methyl, Ethyl, n-und i-Propyl sowie n-, i-, sec-und tert-Butyl. Sie können durch 1 bis 7, vorzugsweise 1 bis 5 und besonders bevorzugt 1 bis 3 (gleichartige oder unterschiedliche) Halogen-Atome substituiert sein. Bevorzugte Halogen-Atome sind Fluor, Chlor, Brom und Iod. Besonders bevorzugt werden Fluor und Chlor. Beispiele, die erwähnt werden können, sind $CF_3$, $CF_2CHCl_2$, $OCF_3$, $OCHF_2$, $OCF_2CHF_2$, $OCF_2CF_3$, $OCH_2CF_3$, $OCF_2CHCl_2$, $OCF_2$, $CHFCF_3$, $SCHF_2$, $SCF_3$, $SCF_2CH_2F$, $SCF_2CF_3$, $SOCF_3$, $SOCH_2CF_3$, $SO_2CF_3$ und $SO_2CH_2CF_3$. Besonders bevorzugt werden $CF_3$, $OCF_3$ und $SCF_3$. Vorzugsweise steht R in der 5-Stellung oder 6-Stellung, besonders bevorzugt in der 6-Stellung, des Benzothiazol-2-yl-oder Benzoxazol-2-yl-Restes.

In den allgemeinen Formeln (I) und (III) bezeichnen X und Y vorzugsweise Halogen.

Die Halogene X und Y in den Formeln (I) und - (III) bedeuten Fluor, Chlor, Brom oder Iod, vorzugsweise Fluor, Chlor und Brom und besonders bevorzugt Fluor und Chlor.

In den allgemeinen Formeln (I) und (II) bezeichnet Z vorzugsweise Schwefel.

W in den allgemeinen Formeln (I) und (III) bedeutet vorzugsweise Sauerstoff.

n in den allgemeinen Formeln (I) und (III) steht vorzugsweise für 0 der 1.

In dem Fall, in dem n 2 bedeutet, befinden sich die Substituenten Y vorzugsweise 2-und 4-Stellung oder in 4-und 6-Stellung des Benzoyl-Restes, und in dem Fall, in dem n 1 bedeutet, befindet sich der Substituent Y vorzugsweise in der 4-, 5-oder 6-Stellung, besonders bevorzugt in der 6-Stellung des Benzoyl-Restes.

Bevorzugte Verbindungen der Formel (I) sind diejenigen, in denen

X und Y unabhängig voneinander ein Halogen-Atom oder $C_1$-$C_4$-Alkyl (vorzugsweise Halogen) bezeichnen,

n 0, 1 oder 2 (vorzugsweise 0 oder 1) bezeichnet,

W Sauerstoff oder Schwefel (vorzugsweise Sauerstoff) bezeichnet,

Z Sauerstoff oder Schwefel (vorzugsweise Schwefel) bezeichnet und

R einen durch Halogen (vorzugsweise Fluor und/oder Chlor) substituierten Rest aus der Gruppe $C_1$-$C_4$-Alkyl, $C_1$-$C_4$-Alkoxy, $C_1$-$C_4$-Alkylthio, $C_1$-$C_4$-Alkyl-sulfinyl und $C_1$-$C_4$-Alkylsulfonyl (vorzugsweise $C_1$-$C_4$-Alkyl, $C_1$-$C_4$-Alkoxy und $C_1$-$C_4$-Alkylthio) bezeichnet.

Bevorzugte Verbindungen der Formel (I) sind diejenigen, in denen X und Y unabhängig voneinander Chlor, Fluor oder Methyl bezeichnen, n 0, 1 oder 2 bezeichnet, Z ein Schwefel-Atom bezeichnet, W ein Sauerstoff-oder Schwefel-Atom bezeichnet und R eine Fluor-substituierte Alkyl-Gruppe mit 1 oder 2 Kohlenstoff-Atomen, eine Fluor-substituierte Alkoxy-Gruppe mit 1 oder 2 Kohlenstoff-Atomen oder eine Fluor-substituierte Alkylthio-Gruppe mit 1 oder 2 Kohlenstoff-Atomen bezeichnet.

Besonders bevorzugte Verbindungen der Formel (I) sind diejenigen, in denen X und Y unabhängig voneinander Chlor oder Fluor bezeichnen, n 0 oder 1 bezeichnet, Z ein Schwefel-Atom bezeichnet, W ein Sauerstoff-Atom bezeichnet und R eine Trifluoromethyl-oder Trifluoro-methoxy-Gruppe bezeichnet.

Darüber hinaus sind zusätzlich zu den vorstehenden Definitionen der bevorzugten oder besonders bevorzugten Verbindungen die am meisten bevorzugten Verbindungen der Formel (I) diejenigen, in denen R in der 6-Stellung des Benzothiazols substituiert ist.

Zu speziellen Beispielen der Verbindungen der Formel (I) gemäß der vorliegenden Erfindung zählen

N-(6-Trifluoromethylbenzothiazol-2-yl)-N'-(2-chlorobenzoyl)harnstoff,

N-(6-Trifluoromethylbenzothiazol-2-yl)-N'-(2,6-difluorobenzoyl)harnstoff,

N-(6-Trifluoromethylbenzothiazol-2-yl)-N'-(2-chloro-6-fluorobenzoyl)harnstoff,

N-(6-Trifluoromethoxybenzothiazol-2-yl)-N'-(2-chloro-benzoyl)harnstoff,

N-(6-Trifluoromethoxybenzothiazol-2-yl)-N'-(2-chloro6-fluorobenzoyl)harnstoff und

N-(6-Trifluoromethoxybenzothiazol-2-yl)-N'-(2,6-difluorobenzoyl)harnstoff,

Wenn beispielsweise 2-Amino-6-trifluoromethylbenzothiazol und 2,6-Difluorobenzoylisocyanat als Ausgangsstoffe in dem obigen Verfahren eingesetzt werden, wird die Reaktion durch das folgende Schema veranschaulicht:

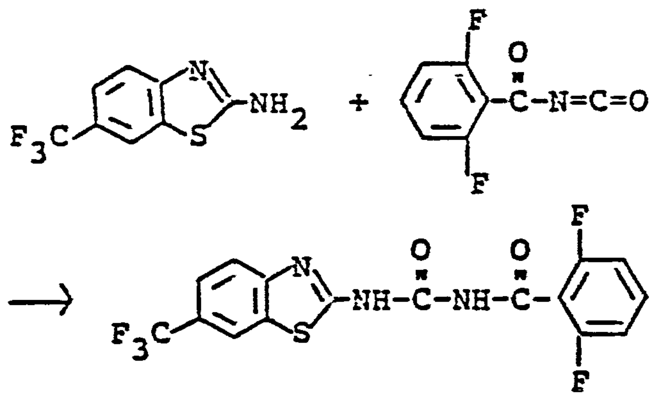

Die Verbindungen der Formel (II) sind bekannte Verbindungen (siehe die US-PS 2 832 761, Zh. Obshch. Khim. 33 (7), 2301-7 (1963) und die EP-Patentveröffentlichung 0 050 551). Zu speziellen Beispielen zählen

2-Amino-6-trifluoromethylbenzothiazol,

2-Amino-6-trifluoromethoxybenzothiazol,

2-Amino-6-difluoromethoxybenzothiazol,

2-Amino-6-(1,1,2,2-tetrafluoroethoxy)benzothiazol,

2-Amino-6-trifluoromethylthiobenzothiazol,

2-Amino-6-(2,2,2-trifluoroethylthio)benzothiazol und

2-Amino-6-pentafluoroethoxybenzothiazol.

Die im Vorstehenden beispielhaft genannten Verbindungen der Formel (II) können aus substituierten Anilinen oder substituierten o-Hydroxyanilinen nach Methoden hergestellt werden, die beispielsweise in J. Chem. Soc. 1969, 268, in der JP-OS 59 679/1984, in der DE-OS 26 01 700, in J. Pharm. Soc., Japan, 73, 1312 (1953) und in J. Am. Chem. Soc. 71, 3417, beschrieben sind.

Die Verbindungen der Formel (III) sind bekannte Verbindungen, die in bekannten Druckschriften bereits vor dem Anmeldedatum der vorliegenden Anmeldung beschrieben wurden, beispielsweise in J. Agr. Food Chem. 21, 348, und in Org. Synthesis, Collective Volume III, p. 735. Zu speziellen Beispielen zählen

2-Chlorobenzoylisocyanat,

2,6-Difluorobenzoylisocyanat,

2-Chloro-6-fluorobenzoylisocyanat,

2-Chlorobenzoylisothiocyanat,

2,6-Difluorobenzoylisothiocyanat und

2-Chloro-6-fluorobenzoylisothiocyanat.

Bei der Durchführung der vorgenannten Verfahren können sämtliche organischen Lösungsmittel als geeignete Verdünnungsmittel dienen.

Zu Beispielen für solche Verdünnungsmittel zählen aliphatische, alicyclische und aromatische Kohlenwasserstoffe (die gegebenenfalls chloriert sein können) wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorobenzol; Ether wie Diethylether, Methylethylether, Diisopropylether, Dibutylether, Propylenoxid, Dioxan und Tetrahydrofuran; Ketone wie Aceton, Methylethylketon, Methylisopropylketon und Methylisobutylketon; Nitrile wie Acetonitril, Propionitril und Acrylnitril; Ester wie Ethylacetat und Amylacetat; Säureamide wie Dimethylformamid und Dimethylacetamid; Sulfone und Sulfoxide wie Dimethylsulfoxid und Sulfolan; und Basen wie Pyridin.

Das vorstehende Verfahren kann in einem weiten Temperaturbereich durchgeführt werden, beispielsweise bei einer Temperatur zwischen etwa -20°C und dem Siedepunkt der Mischung, vorzugsweise zwischen etwa 0°C und etwa 100°C, durchgeführt werden. Die Reaktion wird zweckmäßigerweise unter normalem Atmosphärendruck durchgeführt, jedoch ist es auch möglich, unter erhöhtem oder vermindertem Druck zu arbeiten.

In der Praxis des vorstehenden Verfahrens können die neuen Verbindungen der Formel (1) dadurch erhalten werden, daß vorzugsweise 1 mol der Verbindung der Formel (II) mit etwa 0,8 bis 1,2 mol, besonders bevorzugt 1 bis etwa 1,1 mol, der Verbindung der Formel (III) in einem inerten Lösungsmittel umgesetzt wird.

Die aktiven Verbindungen gemäß der vorliegenden Erfindung zeigen starke pestizide, insbesondere arthropodizide und besonders bevorzugt insektizide Aktivität und können aus diesem Grunde besonders bevorzugt als Insektizide eingesetzt werden. Die aktiven Verbindungen (I) der vorliegenden Erfindung zeigen einen genauen Bekämpfungseffekt gegen Schadinsekten, ohne Schäden bei Kulturpflanzen zu verursachen. Weiterhin können die Verbindungen der vorliegenden Erfindung zur Bekämpfung und Ausrottung eines weiten Spektrums von Schädlingen verwendet werden, darunter saugende Insekten, beißende Insekten und andere Pflanzenparasiten, Schädlingen in Getreidevorräten und gelagerten Körnerfrüchten und gesundheitsgefährdenden Schädlingen.

Beispiele für die Schädlinge sind im Folgenden angegeben.

Insekten der Ordnung Coleoptera

Callosobruchus chinensis,
Sitophilus zeamais,
Tribolium castaneum,
Epilachna vigintioctomaculata,
Agriotes fuscicollis,
Anomala rufocuprea,
Leptinotarsa decemlineata,
Diabrotica spp.,
Monochamus alternatus,
Lissorhoptus oryzophilus und
Lyctus brunneus;

Insekten der Ordnung Lepidoptera

Lymantria dispar,
Malacosoma neustria,
Pieris rapae,
Spodoptera litura,
Mamestra brassicae,
Chilo suppressalis,
Pyrausta nubilalis,
Ephestia cautella,
Adoxophyes orana,
Carpocapsa pomonella,
Agrotis fucosa,
Galleria mellonella,
Heliotis virescens,
Plutella maculipennis und
Phyllocnistis citrella;

Insekten der Ordnung Diptera

Musca domestica,
Aedes aegypti,
Hylemia platura,
Culex pipiens,
Anopheles sinensis und
Culex tritaeniorphynchus.

Auf dem Gebiet der Tierhaltung und Tierzucht sind die neuen Verbindungen gemäß der vorliegenden Erfindung gengenüber verschiedenen schädlichen Tierparasiten wie Insekten (z.B. Gastrophilus spp.) wirksam.

Die aktiven Verbindungen können in gebräuchliche Formulierungen überführt werden, etwa als Lösungen, Emulsionen, Suspensionen, Pulver, Schäume, Pasten, Granulat, Aerosol, mit der aktiven Verbindung imprägnierte natürliche oder synthetische Stoffe, sehr feine Kapseln in polymeren Substanzen, Überzugsmassen zur Verwendung auf Saatgut (Beizmittel) sowie Formulierungen für den Einsatz mit Verbrennungseinrichtungen wie Räucherpatronen, Räucherdosen und Räucherschlangen sowie für die kalte Vernebelung und die warme Vernebelung nach dem Ultra-Low-Volume-Verfahren.

Diese Formulierungen können in bekannter Weise hergestellt werden, beispielsweise durch Vermischen der aktiven Verbindungen mit Streckmitteln, das heißt mit flüssigen oder verflüssigten gasförmigen oder festen Verdünnungsmitteln oder Trägern, gegebenenfalls unter Verwendung grenzflächenaktiver Mittel, das heißt von Emulgatoren und/oder Dispergiermitteln und/oder schaumbildenden Mittteln. Bei Verwendung von Wasser als Streckmittel können organische Lösungsmittel beispielsweise als Hilfslösungsmittel verwendet werden.

Als flüssige Lösungsmittel, Verdünnungsmittel oder Träger hauptsächlich geeignet sind aromatische Kohlenwasserstoffe wie Xylol, Toluol oder Alkylnaphthaline, chlorierte aromatische oder chlorierte aliphatische Kohlenwasserstoffe wie Chlorbenzole, Chloroethylene oder Methylenchlorid, aliphatische Kohlenwasserstoff wie Cyclohexan

oder Paraffine, beispielsweise Mineralöl-Fraktionen, Alkohole wie Butanol oder Glycol sowie deren Ether und Ester, Ketone wie Aceton, Methylethylketon, Methylisobutylketon oder Cyclohexanon oder stark polare Lösungsmittel wie Dimethylformamid und Dimethyl-sulfoxid sowie auch Wasser.

Unter verflüssigten gasförmigen Verdünnungsmitteln oder Trägern sind Flüssigkeiten zu verstehen, die bei normaler Temperatur und normalem Druck gasförmig wären, beispielsweise Aerosol-Treibmittel wie halogenierte Kohlenwasserstoffe sowie Butan, Propan, Stickstoff und Kohlenstoffdioxid.

Als feste Träger verwendbar sind gemahlene natürliche Minerale wie Kaoline, Tone, Talkum, Kreide, Quarz, Attapulgit, Montmorillonit oder Diatomeenerde und gemahlene synthetische Minerale wie hochdisperse Kieselsäure, Aluminiumoxid und Silicate. Als feste Träger für Granulat können zerkleinerte und fraktionierte Natursteinmaterialien verwendet werden, etwa Calcit, Marmor, Bimsstein, Sepiolith und Dolomit sowie synthetisches Granulat aus anorganischen und organischen Mehlen und Granulat aus organischem Material wie Sägemehl, Koskosnußschalen, Maiskolben und Tabakstengel.

Als emulgierende und/oder schaumbildende Mittel können nicht-ionische und anionische Emulgatoren wie Polyoxyethylenfettsäureester, Polyoxyethylenfettalkoholether, beispielsweise Alkylarylpolyglycol-Ether, Alkylsulfonate, Alkylsulfate, Arylsulfonate sowie Albumin-Hydroly-seprodukte verwendet werden. Zu Dispergiermitteln zählen beispielsweise Ligninsulfit-Ablaugen und Methyl-cellulose.

Haftmittel wie Carboxymethylcellulose und natürliche und synthetische Polymere in Form von Pulvern, Granulat oder Latices wie Gummi arabicum, Polyvinylalkohol und Polyvinylacetat können bei der Formulierung verwendet werden.

Es ist auch möglich, farbgebende Mittel, etwa anorganische Pigmente wie beispielsweise Eisenoxid, Titanoxid und Preußisch Blau und organische Farbstoffe wie Alizarin-Farbstoffe, Azo-Farbstoffe oder Metallphthalocyanin-Farbstoffe, sowie Spuren-Nährstoffe wie Salze von Eisen, Mangan, Bor, Kupfer, Cobalt, Molybdän und Zink zu verwenden.

Die Formulierungen enthalten im allgemeinen 0,1 bis 95 Gew.-%, vorzugsweise 0,5 bis 90 Gew.-% der aktiven Verbindung.

Die aktiven Verbindungen gemäß der Erfindung können in ihren handelsüblichen Formulierungen oder den aus diesen Formulierungen hergestellten Anwendungsformen im Gemisch mit anderen aktiven Verbindungen vorliegen, etwa mit Insektiziden, Ködern, Sterilisationsmitteln, Akariziden, Nematiziden, Fungiziden, Wachstumsregulatoren oder Herbiziden. Zu den Insektiziden gehören beispielsweise Phosphate, Carbamate, Carboxylate, chlorierte Kohlenwasserstoffe, Phenylharnstoffe und von Mikroorganismen erzeugte Substanzen.

Die aktiven Verbindungen gemäß der vorliegenden Erfindung können weiterhin in ihren handelsüblichen Formulierungen oder den aus diesen Formulierungen hergestellten Anwendungsformen im Gemisch mit synergistischen Mitteln vorliegen. Synergistische Mittel sind Verbindungen, die die Wirkung der aktiven Verbindungen steigern, ohne daß es für das zugesetzte synergistische Mittel erforderlich ist, selbst aktiv zu sein.

Der Gehalt der aktiven Verbindung in den aus den im Handel erhältlichen Formulierungen hergestellten Anwendungsformen kann innerhalb weiter Grenzen variieren. Die Konzentration der aktiven Verbindung in den Anwendungsformen kann 0,0000001 bis 100 Gew.-% betragen und liegt vorzugsweise zwischen 0,0001 und 1 Gew.-%.

Die Verbindungen werden in üblicher Weise in einer den Anwendungsformen angemessenen Form zur Anwendung gebracht.

Bei der Verwendung gegen Hygiene-Schädlinge und Schädlinge in Produkt-Vorräten zeichnen sich die aktiven Verbindungen durch eine hervorragende Rückstandswirkung auf Holz und Ton sowie eine gute Beständigkeit gegen Alkali auf gekalkten Unterlagen aus.

Die folgenden Beispiele erläutern die vorliegende Erfindung im einzelnen. Es sei jedoch darauf hingewiesen, daß die vorliegende Erfindung nicht allein auf diese speziellen Beispiele beschränkt ist.

Herstellungsbeispiele

Beispiel 1

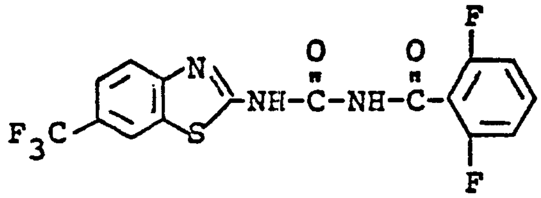

(Verbindung Nr. 1)

2-Amino-6-trifluoromethylbenzothiazol (5,58 g) wurde in 20 ml trockenem Tetrahydrofuran gelöst, und unter Rühren und Kühlung wurde 2,6-Difluorobenzoylisocyanat (4,68 g) tropfenweise zugesetzt. Die Reaktionsmischung wurde 8 h bei Raumtemperatur gerührt, und das Lösungsmittel wurde unter vermindertem Druck abgedampft. Der Rückstand wurde aus Ethanol umkristallisiert, wonach 8,70 g des gewünschten N-(6-Trifluoromethylbenzothiazol-2-yl)-N'-(2,6-difluorobenzoyl)harnstoffs erhalten wurden. Schmp. oberhalb von 300°C.

Beispiel 2

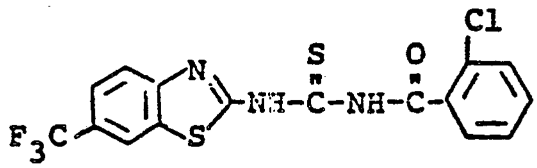

(Verbindung Nr. 2)

Kaliumthiocyanat (0,49 g) wurde in 10 ml Acetonitril gelöst, und unter Kühlung wurde 2-Chlorobenzoylchlorid (0,88 g) hinzugefügt. Die Mischung wurde zur Herstellung von 2-Chlorobenzoylisothiocyanat 1 h bei 60°C gerührt. Ohne Isolierung des Produkts wurde die Reaktionsmischung auf Raumtemperatur gekühlt, und 5 ml einer Tetrahydrofuran-Lösung von 1,09 g 2-Amino-6-trifluoromethylbenzothiazol wurden zugegeben. Die Mischung wurde 4 h bei 50°C gerührt. Das Lösungsmittel wurde unter vermindertem Druck abgedampft. Nach Zusatz von Wasser wurde der Rückstand durch Filtration gesammelt, mit Wasser gewaschen und getrocknet. Das Rohprodukt wurde aus Ethanol umkristallisiert, wonach der gewünschte N-(6-Trifluoromethylbenzothiazol-2-yl)-N'-(2-chlorobenzoyl)thioharnstoff (0,90 g) erhalten wurde. Schmp. 225-227°C.

Verbindungen, die nach den gleichen Verfahrensweisen hergestellt wurden, wie sie in den vorstehenden Beispielen beschrieben sind, sind in der Tabelle 1 aufgeführt. Die Bezeichnung "-"in der "$Y_n$" überschriebenen Spalte bedeutet die Abwesenheit eines Substituenten.

Tabelle 1

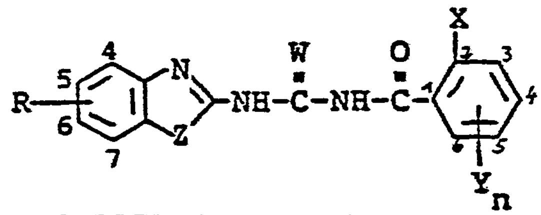

| Verbindung Nr. | R | Z | W | X | $Y_n$ | Physikal. Konstante |
|---|---|---|---|---|---|---|
| 3 | $6-CF_3$ | S | O | Cl | - | Schmp. >300°C |
| 4 | $6-CF_3$ | S | O | Cl | 6-F | Schmp. >300°C |
| 5 | $6-CF_3$ | S | S | F | 6-F | Schmp. 203-205°C |
| 6 | $6-CF_3$ | S | S | Cl | 6-F | Schmp. 206-210°C |
| 7 | $6-CF_3$ | S | S | Cl | 6-Cl | Schmp. 216-220°C |

Tabelle 1 - Fortsetzung

| Verbindung Nr. | R | Z | W | X | $Y_n$ | Physikal. Konstante |
|---|---|---|---|---|---|---|
| 8 | $6-CF_3$ | S | O | Cl | 6-Cl | Schmp. >300°C |
| 9 | $6-CF_3$ | O | O | Cl | - | |
| 10 | $6-OCF_3$ | S | O | Cl | - | Schmp. 210-211°C |
| 11 | $6-SCF_3$ | S | O | Cl | - | Schmp. 216-219°C |
| 12 | $6-\overset{O}{\overset{\|}{S}}-CF_3$ | S | O | Cl | - | |
| 13 | $6-\underset{O}{\underset{\|}{\overset{O}{\overset{\|}{S}}}}-CF_3$ | S | O | Cl | - | |
| 14 | $6-OCHF_2$ | S | O | Cl | - | |
| 15 | $6-OCHF_2$ | S | S | Cl | - | |
| 16 | $6-SCH_2CF_3$ | S | O | Cl | - | |
| 17 | $6-OCF_2CF_3$ | S | O | Cl | - | |
| 18 | $6-OCF_2CHF_2$ | S | O | Cl | - | |
| 19 | $6-OCF_2CHCl_2$ | S | O | Cl | - | |
| 20 | $6-OCF_2CHFCF_3$ | S | O | Cl | - | |
| 21 | $6-OCF_3$ | S | O | Cl | 6-Cl | |
| 22 | $6-OCF_3$ | S | O | F | 6-F | Schmp. 204-206°C |
| 23 | $6-OCF_3$ | S | O | $-CH_3$ | - | |
| 24 | $6-OCF_3$ | S | O | $-C_2H_5$ | - | |
| 25 | $6-OCF_3$ | S | O | F | $4,6-F_2$ | |
| 26 | $6-OCF_3$ | S | O | Cl | $6-CH_3$ | |
| 27 | $6-OCF_3$ | S | O | Cl | 6-F | Schmp. 193-195°C |

Verwendungsbeispiele

Vergleichs-Verbindung E-1:

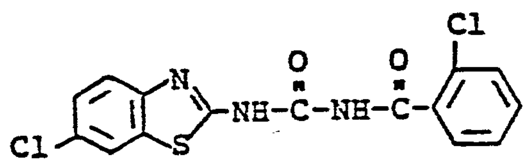

(die in der JP-OS 124 265/1978 beschriebene Verbindung)

Beispiel 3

Test mit Larven von Spodoptera litura:

**<u>Test mit Larven von Spodoptera litura:</u>**

**Herstellung einer Test-Chemikalie:**

| | | |
|---|---|---|
| **Lösungsmittel:** | **3 Gew.-Teile** | **Dimethylformamid** |
| **Emulgator:** | **1 Gew.-Teil** | **Polyoxyethylen-alkylphenylether** |

Zur Herstellung eines geeigneten Wirkstoff-Präparats wurde 1 Gew.-Teil der aktiven Verbindung mit dem Lösungsmittel und dem Emulgator in den oben angegebenen Mengen vermischt. Die Mischung wurde mit Wasser auf eine vorher festgesetzte Konzentration verdünnt.

Test-Verfahren:

Kohlblätter wurden in die wäßrige Verdünnung der aktiven Verbindung mit der vorbestimmten Konzentration getaucht. Nach dem Luft-Trocknen der Chemikalie wurden die Kohlblätter in Petrischalen von 9 cm Durchmesser gelegt. 5 Larven im 3. Stadium von Spodoptera litura wurden in der Petrischale ausgesetzt, und die Schalen wurden in einem thermostatisierten Raum von 28°C gestellt. 7 d danach wurde das Vernichtungsverhältnis berechnet.

Die mit typischen Verbindungen erhaltenen Ergebnisse sind in Tabelle 2 aufgeführt.

Tabelle 2

| Verbindung Nr. | Wirkstoff-Konzentration ppm | Vernichtungsverhältnis % |
|---|---|---|
| 2 | 2 | 100 |
| 3 | 2 | 100 |
| 10 | 2 | 100 |
| Vergleichs-Verbindung E-1 | 10 | 100 |
| | 2 | 0 |

Beispiel 4

Test mit Larven von Plutella maculipennis:

Test-Verfahren:

Kohlblätter wurden in die wäßrige Verdünnung der aktiven Verbindung mit der vorbestimmten Konzentration getaucht, die wie in Beispiel 3 hergestellt wurde. Nach dem Luft-Trocknen der Chemikalie wurden die Kohlblätter in Petrischalen von 9 cm Durchmesser gelegt. 10 Larven von Plutella maculipennis wurden in der Petrischale ausgesetzt, und die Schalen wurden in einen thermostatisierten Raum von 23°C gestellt. 7 d danach wurde das Vernichtungsverhältnis berechnet.

Die mit typischen Verbindungen erhaltenen Ergebnisse sind in Tabelle 3 aufgeführt.

Tabelle 3

| Verbindung Nr. | Wirkstoff-Konzentration ppm | Vernichtungs-verhältnis % |
|---|---|---|
| 1 | 2 | 100 |
| 3 | 2 | 100 |
| 4 | 2 | 100 |
| Vergleichs-Verbindung E-1 | 40 | 100 |
| | 20 | 0 |
| | 2 | 0 |

**Ansprüche**

1. Benzoylharnstoff der Formel (I)

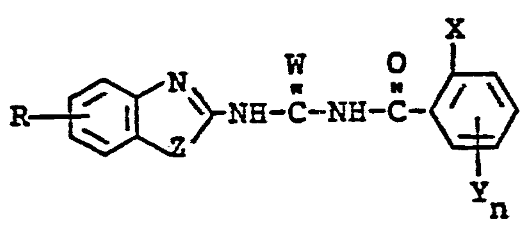

(I)

in der

X und Y unabhängig voneinander ein Halogen-Atom oder eine Niederalkyl-Gruppe bezeichnen,

n 0, 1 oder 2 bezeichnet,

Z und W unabhängig voneinander ein Sauerstoff- oder Schwefel-Atom bezeichnen und

R eine Halogen-substituierte Niederalkyl-Gruppe,

eine Halogen-substituierte Niederalkoxy-Gruppe,

eine Halogen-substituierte Niederalkylthio-Gruppe,

eine Halogen-substituierte Niederalkylsulfinyl-Gruppe oder eine Halogen-substituierte Niederalkylsulfonyl-Gruppe bezeichnet.

2. Verbindungen der Formel (I) nach Anspruch 1, dadurch gekennzeichnet, daß

X und Y unabhängig voneinander Halogen oder $C_1$-$C_4$-Alkyl bezeichnen

n 0 oder 1 bezeichnet,

W Sauerstoff oder Schwefel bezeichnet,

Z Sauerstoff oder Schwefel bezeichnet und

R einen durch Halogen substituierten Rest aus der Gruppe $C_1$-$C_4$-Alkyl, $C_1$-$C_4$-Alkoxy, $C_1$-$C_4$-Alkylthio, $C_1$-$C_4$-Alkylsulfinyl und $C_1$-$C_4$-Alkylsulfonyl bezeichnet.

3. Verbindungen der Formel (I) nach Anspruch 1, dadurch gekennzeichnet, daß

X und Y jeweils Chlor, Fluor oder Methyl sind, n 0, 1 oder 2 ist, Z ein Schwefel-Atom ist, W ein Sauerstoff-oder Schwefel-Atom ist und R eine Fluor-substituierte Alkyl-Gruppe mit 1 oder 2 Kohlenstoff-Atomen, eine Fluor-substituierte Alkoxy-Gruppe mit 1 oder 2 Kohlenstoff-Atomen oder eine Fluor-substituierte Alkylthio-Gruppe mit 1 oder 2 Kohlenstoff-Atomen ist.

4. Verbindungen der Formel (I) nach Anspruch 1, dadurch gekennzeichnet, daß

X und Y Chlor oder Fluor sind, n 0 oder 1 ist, Z ein Schwefel-Atom ist, W ein Sauerstoff-Atom ist und R eine Trifluoromethyl-oder Trifluoromethoxy-Gruppe ist.

5. Verbindungen nach Ansprüchen 1 bis 4, ausgewählt aus den Verbindungen der Formeln

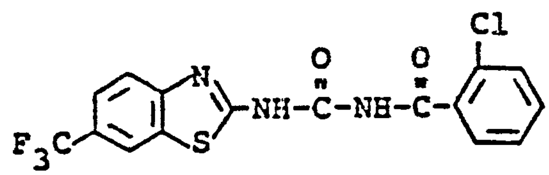

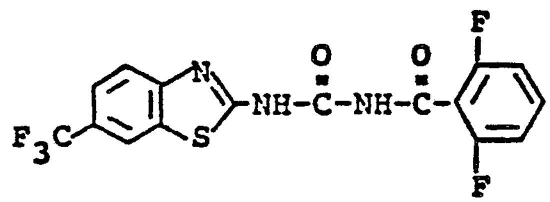

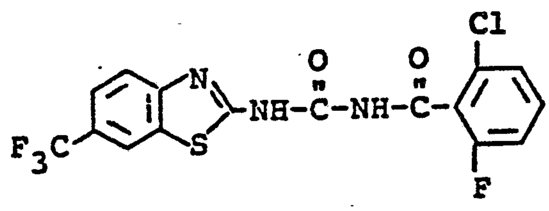

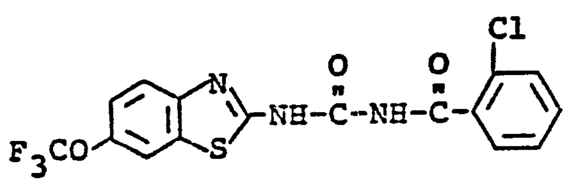

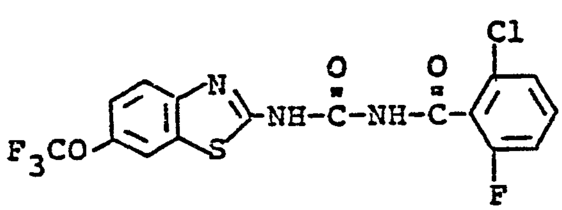

und

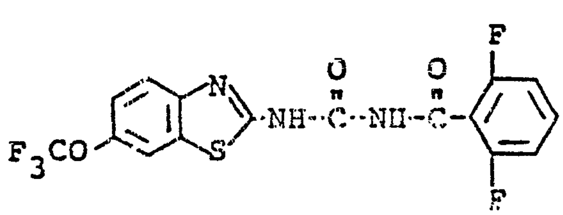

6. Verfahren zur Herstellung neuer Benzoylharnstoffe der Formel (I)

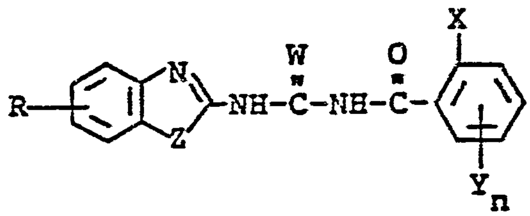

(I)

in der

X und Y unabhängig voneinander ein Halogen-Atom oder eine Niederalkyl-Gruppe bezeichnen,

n 0, 1 oder 2 bezeichnet,

Z und W unabhängig voneinander ein Sauerstoff- oder Schwefel-Atom bezeichnen und

R eine Halogen-substituierte Niederalkyl-Gruppe,

eine Halogen-substituierte Niederalkoxy-Gruppe,

eine Halogen-substituierte Niederalkylthio-Gruppe,

eine Halogen-substituierte Niederalkylsulfinyl-Gruppe oder eine Halogen-substituierte Niederalkylsulfonyl-Gruppe bezeichnet,

dadurch gekennzeichnet, daß die Verbindungen der Formel (II)

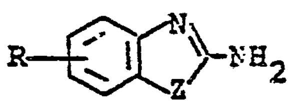

(II)

in der Z und R die angegebenen Bedeutungen haben, mit Verbindungen der Formel (III)

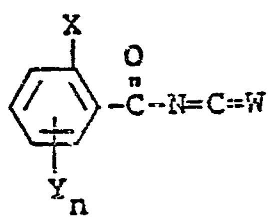

(III)

in der X, Y, n und W die angegebenen Bedeutungen haben, umgesetzt werden, gegebenenfalls in Gegenwart inerter Lösungsmittel.

7. Pestizide Mittel, dadurch gekennzeichnet, daß die wenigstens der Benzoylharnstoffe der Formel - (I) nach Ansprüchen 1 bis 6 enthalten.

8. Verfahren zur Bekämpfung von Schädlingen, vorzugsweise von Insekten, dadurch gekennzeichnet, daß man Benzoylharnstoffe der Formel (I) nach Ansprüchen 1 bis 6 auf Schädlinge, vorzugsweise Insekten und/oder deren Lebensraum einwirken läßt.

9. Verwendung von Benzoylharnstoffen der Formel (I) nach Ansprüchen 1 bis 6 zur Bekämpfung von Schädlingen, vorzugsweise von Insekten.

10. Verfahren zur Herstellung von pestiziden Mitteln, dadurch gekennzeichnet, daß Benzoylharnstoffe der Formel (I) nach Ansprüchen 1 bis 6 mit Streckmitteln und/oder grenzflächenaktiven Mitteln vermischt werden.